# EUROPEAN PATENT APPLICATION

(11) **EP 3 524 143 A1**
(43) Date of publication of application: **14.08.2019**
(21) Application number: 17865115.4
(22) Date of filing: 10.01.2017
(51) Int. Cl.: A61B 5/021

(54) **METHOD AND APPARATUS FOR WEARABLE DEVICES TO MEASURE BLOOD PRESSURE**

(30) Priority: 28.10.2016 CN 201610968184
(71) Applicant: Huawei Technologies Co., Ltd., Longgang District Shenzhen, Guangdong 518129 (CN)
(72) Inventor: SUN, Shiyou, Shenzhen Guangdong 518129 (CN); WANG, Zhiyong, Shenzhen Guangdong 518129 (CN); NI, Xiaodong, Shenzhen Guangdong 518129 (CN); XI, Yi, Shenzhen Guangdong 518129 (CN)
(74) Representative: Gill Jennings & Every LLP
(86) International application number: PCT/CN2017/070781
(87) International publication number: WO 2018/076555

(57) **Abstract**

A blood pressure measurement method and apparatus for a wearable device are provided. The method includes: receiving, by a first wearable device, a blood pressure measurement instruction; sending, by the first wearable device, the received blood pressure measurement instruction to a second wearable device; receiving, by the first wearable device, feedback information sent by the second wearable device; starting, by the first wearable device, to collect the first blood pressure reference data; receiving, by the first wearable device, the second blood pressure reference data that is collected by the second wearable device and that is of first specified duration; and calculating, by the first wearable device, blood pressure based on the first blood pressure reference data of the first specified duration and the second blood pressure reference data of the first specified duration. With the foregoing method, blood pressure can be measured conveniently, continuously, and accurately, and user experience is improved.

## Description

This application claims priority to Chinese Patent Application No. 201610968184.X, filed with the Chinese Patent Office on October 28, 2016 and entitled "BLOOD PRESSURE MEASUREMENT METHOD FOR WEARABLE DEVICE", which is incorporated herein by reference in its entirety.

### TECHNICAL FIELD

Embodiments of the present invention relate to the field of wearable device technologies, and in particular, to a blood pressure measurement method and apparatus for a wearable device.

### BACKGROUND

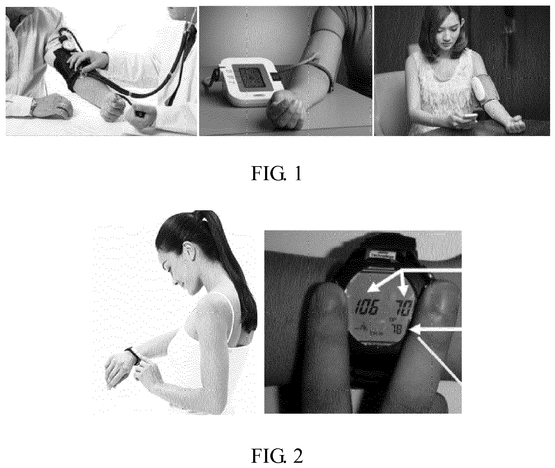

An existing blood pressure measurement method mainly includes the following two measurement methods. A first method is a cuff measurement method in which a conventional mercury sphygmomanometer or pressure detection device is used to perform measurement. As shown in FIG. 1, when the method is used to perform measurement, a cuff is placed at an upper arm, and blood pressure (Blood Pressure, BP) is measured by listening to a vascular sound through air compression. When the cuff is used to measure the blood pressure, there is a slight discomfort feeling in a compression process, operation is not convenient either, and the blood pressure cannot be continuously measured anytime anywhere. In a second method, blood pressure measurement is performed by using a wearable blood pressure measurement device by performing a joint operation with left and right hands. As shown in FIG. 2, in the method, an operation needs to be performed at a wrist by using two hands, to separately obtain a photoplethysmogram (Photoplethysmogram, PPG) signal and an electrocardiograph (Electrocardiograph, ECG) signal of a human body through measurement, and then calculate blood pressure. However, the ECG signal obtained at the wrist through measurement has relatively poor quality, and accuracy of the calculated blood pressure is relatively low. In addition, due to a limitation from an operation manner in which one hand is placed on the other hand, the blood pressure cannot be continuously measured anytime anywhere in the method either. In particular, when blood pressure in a sleep state and a motion state needs to be measured, the method has higher difficulty in implementing measurement.

In conclusion, how to measure blood pressure accurately is a problem that needs to be resolved currently.

### SUMMARY

An objective of embodiments of the present invention is to provide a blood pressure measurement method and apparatus for a wearable device, to improve blood pressure measurement accuracy.

According to a first aspect, a blood pressure measurement method for a wearable device is provided, where the method includes: generating, by a first wearable device based on triggering of a user or a second wearable device, a blood pressure measurement instruction that is used to instruct the second wearable device to perform blood pressure measurement; sending, by the first wearable device, the received blood pressure measurement instruction to the second wearable device; receiving, by the first wearable device, feedback information sent by the second wearable device, where the feedback information is used to notify the first wearable device that the second wearable device successfully receives the blood pressure measurement instruction; after receiving the feedback information, starting, by the first wearable device, to collect the first blood pressure reference data; receiving, by the first wearable device, the second blood pressure reference data that is collected by the second wearable device in first specified duration; and calculating, by the first wearable device, blood pressure based on the first blood pressure reference data that is collected by the first wearable device in the first specified duration and the second blood pressure reference data that is collected by the second wearable device in the first specified duration, where the first wearable device is worn at a part that is of a human body and at which the first blood pressure reference data can be obtained through measurement, and the second wearable device is worn at a part that is of the human body and at which the second blood pressure reference data can be obtained through measurement.

In this embodiment of the present invention, the first wearable device and the second wearable device simultaneously start to collect reference data at different parts of the human body, to calculate the blood pressure, so that blood pressure calculation accuracy is improved.

In a possible design, the first blood pressure reference data may be a photoplethysmogram PPG signal or an electrocardiograph ECG signal, and the second blood pressure reference data may be an ECG signal or a PPG signal; and when the first blood pressure reference data is a PPG signal, the second blood pressure reference data is an ECG signal; or when the first blood pressure reference data is an ECG signal, the second blood pressure reference data is a PPG signal. The PPG signal may be collected at a pulse of the human body, and the ECG signal may be collected at the chest or an upper arm of the human body.

In this embodiment of the present invention, the first wearable device or the second wearable device may collect the PCG signal or the ECG signal, and the first wearable device and the second wearable device may be conveniently worn at each part of the human body, so that continuous measurement is implemented, and the wearable device is used more flexibly.

In a possible design, the method further includes: if the first wearable device does not receive, within a second specified time, the feedback information sent by the second wearable device, re-sending, by the first wearable device, the blood pressure instruction to the second wearable device.

In this embodiment of the present invention, only after receiving the feedback information sent by the second wearable device, the first wearable device starts to collect reference data, so that it is ensured that the first wearable device and the second wearable device simultaneously collect data, and only when calculation is performed based on the collected reference data, high blood pressure calculation accuracy can be ensured.

In a possible design, a specified temperature compensated crystal oscillator (Temperature Compensated Crystal Oscillator, TCXO) is installed in each of the first wearable device and the second wearable device, to ensure that in the first specified duration, a frequency offset delay of the first wearable device and the second wearable device falls within an allowable deviation range, so that it is ensured that a deviation is small when the first wearable device and the second wearable device collect reference data in a long time.

According to a second aspect, a blood pressure measurement method for a wearable device is provided, where the method includes: receiving, by a second wearable device, a blood pressure measurement instruction sent by a first wearable device; sending, by the second wearable device, feedback information to the first wearable device, where the feedback information is used to notify the first wearable device that the second wearable device successfully receives the blood pressure measurement instruction; starting, by the second wearable device third specified duration later after the feedback information is sent, to collect the second blood pressure reference data in first specified duration; and sending, by the second wearable device, the second blood pressure reference data collected in the first specified duration to the first wearable, where the blood pressure measurement instruction is used to instruct the first wearable device and the second wearable device to perform blood pressure measurement, the first wearable device is worn at a part that is of a human body and at which first blood pressure reference data can be obtained through measurement, and the second wearable device is worn at a part of the human body and at which the second blood pressure reference data can be obtained through measurement.

In this embodiment of the present invention, the third specified duration later after the feedback message is sent, the second wearable device and the first wearable device simultaneously start to collect reference data at different parts of the human body, to calculate blood pressure, so that blood pressure calculation accuracy is improved.

In a possible design, the second blood pressure reference data may be a photoplethysmogram PPG signal or an electrocardiograph ECG signal.

According to a third aspect, a blood pressure measurement apparatus for a wearable device is provided, where the apparatus includes: a receiving module, configured to generate a blood pressure measurement instruction, where the blood pressure measurement instruction is used to instruct a second wearable device to perform blood pressure measurement, and the first wearable device is worn at a part that is of a human body and at which first blood pressure reference data can be obtained through measurement; a sending module, configured to send the blood pressure measurement instruction to the second wearable device, where the receiving module is further configured to receive feedback information sent by the second wearable device, where the feedback information is used to notify the first wearable device that the second wearable device successfully receives the blood pressure measurement instruction; a collection module, configured to collect the first blood pressure reference data based on the feedback information, where a time of collecting the first blood pressure reference data is first specified duration, where the receiving module is further configured to receive the second blood pressure reference data that is collected by the second wearable device in the first specified duration; and a processing module, configured to calculate blood pressure based on the first blood pressure reference data and the second blood pressure reference data.

In this embodiment of the present invention, the first wearable device and the second wearable device simultaneously start to collect reference data at different parts of the human body, to calculate the blood pressure, so that blood pressure calculation accuracy is improved.

In a possible design, the first blood pressure reference data may be a photoplethysmogram PPG signal or an electrocardiograph ECG signal, and the second blood pressure reference data may be an ECG signal or a PPG signal; and when the first blood pressure reference data is a PPG signal, the second blood pressure reference data is an ECG signal; or when the first blood pressure reference data is an ECG signal, the second blood pressure reference data is a PPG signal.

In a possible design, the sending module is further configured to: if the receiving module does not receive, within a second specified time, the feedback information sent by the second wearable device, re-send the blood pressure instruction to the second wearable device.

In a possible design, the blood pressure measurement instruction generated by the receiving module may be triggered by a user, or may be triggered by the second wearable device.

According to a fourth aspect, a blood pressure measurement apparatus for a wearable device is provided, where the apparatus includes: a receiving module, configured to receive a blood pressure measurement instruction sent by a first wearable device, where the blood pressure measurement instruction is used to instruct the second wearable device to perform blood pressure measurement, and the second wearable device is worn at a part that is of a human body and at which second blood pressure reference data can be obtained through measurement; a sending module, configured to send feedback information to the first wearable device, where the feedback information is used to notify the first wearable device that the receiving module successfully receives the blood pressure measurement instruction; and a collection module, configured to start, third specified duration later after the sending module sends the feedback information, to collect the second blood pressure reference data in first specified duration, where the sending module is further configured to send the second blood pressure reference data collected in the first specified duration to the first wearable device.

In this embodiment of the present invention, the third specified duration later after the feedback message is sent, the second wearable device and the first wearable device simultaneously start to collect reference data at different parts of the human body, to calculate blood pressure, so that blood pressure calculation accuracy is improved.

In a possible design, the second blood pressure reference data may be a photoplethysmogram PPG signal or an electrocardiograph ECG signal.

According to a fifth aspect, an embodiment of the present invention provides a first wearable device for measuring blood pressure, including a first processor and a first memory connected to the first processor, where
the first memory is configured to store program code executed by the first processor; and
the first processor is configured to execute the program code stored by the first memory, to perform the following processes:
receiving, by the first wearable device, a blood pressure measurement instruction, where the blood pressure measurement instruction is used to instruct the first wearable device and a second wearable device to perform blood pressure measurement, and the first wearable device is worn at a part that is of a human body and at which first blood pressure reference data can be obtained through measurement; sending, by the first wearable device, the received blood pressure measurement instruction to the second wearable device, where the second wearable device is worn at a part that is of the human body and at which second blood pressure reference data can be obtained through measurement; receiving, by the first wearable device, feedback information sent by the second wearable device, where the feedback information is used to notify the first wearable device that the second wearable device successfully receives the blood pressure measurement instruction; starting, by the first wearable device, to collect the first blood pressure reference data; receiving, by the first wearable device, the second blood pressure reference data that is collected by the second wearable device in first specified duration; and calculating, by the first wearable device, blood pressure based on the first blood pressure reference data that is collected by the first wearable device in the first specified duration and the second blood pressure reference data that is collected by the second wearable device in the first specified duration.

According to a sixth aspect, an embodiment of the present invention provides a second wearable device for measuring blood pressure, including a second processor and a memory connected to the processor, where
the second memory is configured to store program code executed by the second processor; and
the second processor is configured to execute the program code stored by the second memory, to perform the following processes:
receiving, by the second wearable device, a blood pressure measurement instruction sent by a first wearable device, where the blood pressure measurement instruction is used to instruct the first wearable device and the second wearable device to perform blood pressure measurement, the first wearable device is worn at a part of a human body and at which first blood pressure reference data can be obtained through measurement, the second wearable device is worn at a part that is of the human body and at which second blood pressure reference data can be obtained through measurement; sending, by the second wearable device, feedback information to the first wearable device, where the feedback information is used to notify the first wearable device that the second wearable device successfully receives the blood pressure measurement instruction; starting, by the second wearable device third specified duration later after the feedback information is sent, the second blood pressure reference data in first specified duration; and sending, by the second wearable device, the second blood pressure reference data collected in the first specified duration to the first wearable device.

In the embodiments of the present invention, the first wearable device and the second wearable device simultaneously start to collect reference data at different parts of the human body, to calculate the blood pressure, so that blood pressure calculation accuracy is improved.

### BRIEF DESCRIPTION OF DRAWINGS

FIG. 1 is a schematic diagram of a blood pressure measurement method for a wearable device according to an embodiment of the present invention;
FIG. 2 is a schematic diagram of another blood pressure measurement method for a wearable device according to an embodiment of the present invention;
FIG. 3 is a schematic diagram of still another blood pressure measurement method for a wearable device according to an embodiment of the present invention;
FIG. 4 is a schematic diagram of yet another blood pressure measurement method for a wearable device according to an embodiment of the present invention;
FIG. 5A and FIG. 5B are flowcharts of a blood pressure measurement method for a wearable device according to an embodiment of the present invention;
FIG. 6 is a schematic structural diagram of a blood pressure measurement apparatus for a wearable device according to an embodiment of the present invention;
FIG. 7 is a schematic structural diagram of another blood pressure measurement apparatus for a wearable device according to an embodiment of the present invention;
FIG. 8 is a schematic diagram of a hardware structure of a first wearable device for measuring blood pressure according to an embodiment of the present invention; and
FIG. 9 is a schematic diagram of a hardware structure of a second wearable device for measuring blood pressure according to an embodiment of the present invention.

### DESCRIPTION OF EMBODIMENTS

The following further describes the embodiments of the present invention in detail with reference to this specification. It should be understood that the specific embodiments described herein are only used to explain the present invention but are not intended to limit the present invention.

As people pay much attention to physical health, growing importance is also attached to a blood pressure value, especially blood pressure in a sleep state and a motion state. Therefore, how to accurately measure the blood pressure is a problem that needs to be resolved currently.

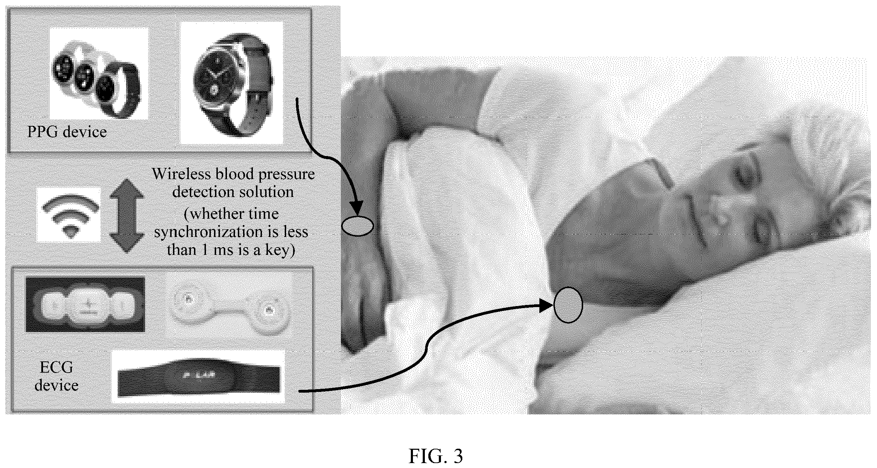

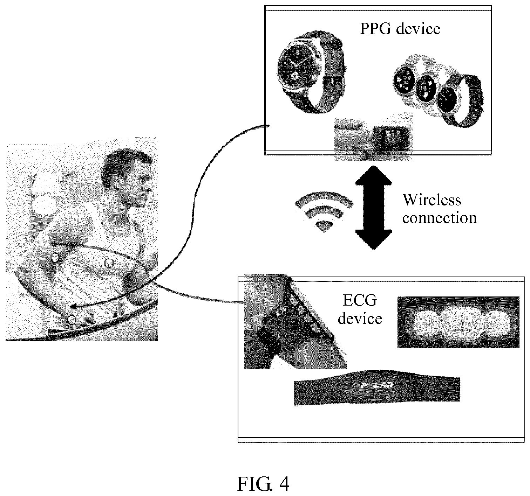

The embodiments of the present invention provide wearable devices shown in FIG. 3 and FIG. 4. In FIG. 3, a PPG device and an ECG device respectively monitor blood pressure at a wrist and the heart in a sleep scenario. The PPG device is worn at the wrist like a watch or a band, and the ECG device may be attached to the chest like a chest patch. In FIG. 4, a PPG device and an ECG device respectively monitor blood pressure at a wrist and the heart in a motion scenario. The PPG device is worn at the wrist like a watch or band, and the ECG device may be put on the chest like a heart rate strap or a chest patch, or may be worn on an arm like an arm band, so that continuous monitoring is implemented.

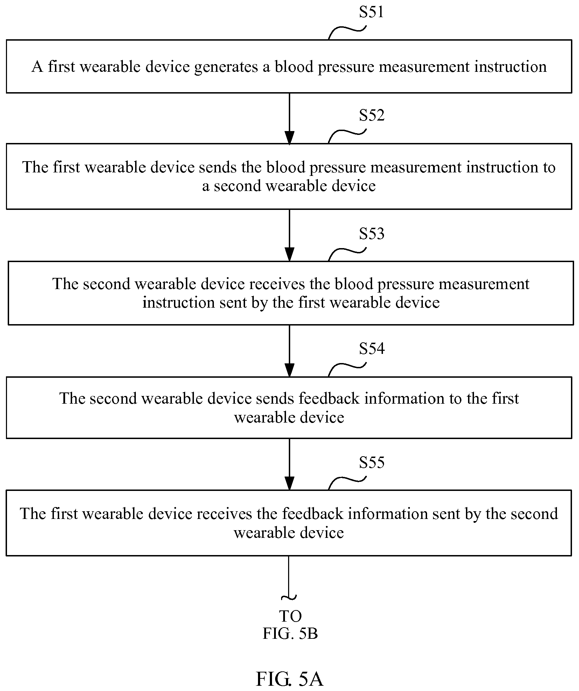

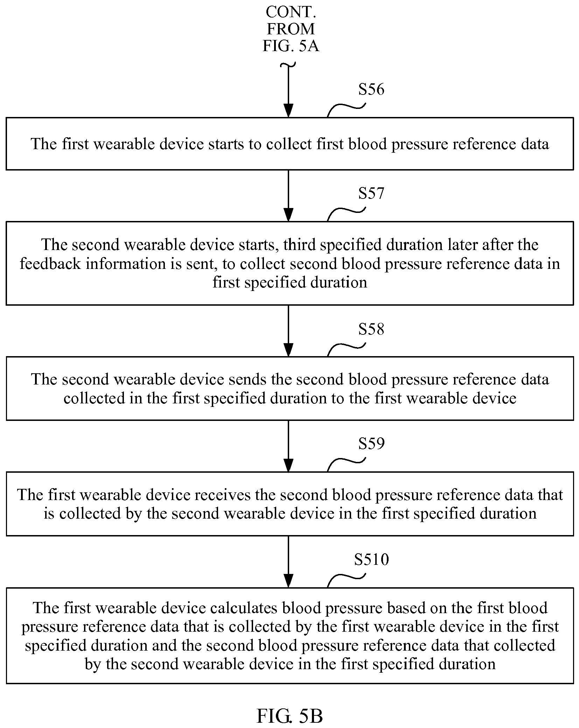

In the embodiments of the present invention, a specific embodiment is used to describe a method for measuring blood pressure. As shown in FIG. 5A and FIG. 5B, it is assumed that a first wearable device measures a PPG signal, a second wearable device measures an ECG signal, a user triggers a blood pressure measurement instruction, and the first wearable device successfully receives feedback information sent by the second wearable device. The following processes are included.

S51. The first wearable device generates a blood pressure measurement instruction, where the blood pressure measurement instruction is used to instruct the second wearable device to perform blood pressure measurement, and the first wearable device is worn at a part that is of a human body and at which first blood pressure reference data can be obtained through measurement.

Optionally, the blood pressure measurement instruction may be triggered by a user, or may be triggered by the second wearable device. The first blood pressure reference data may be a PPG signal or an ECG signal. When the first blood pressure reference data is a PPG signal, the first wearable device may be worn at a part, such as a wrist or an ankle, that is of the human body and at which the PPG signal can be obtained through measurement. When the first blood pressure reference data is an ECG signal, the first wearable device may be worn at a part, such as the chest or an upper arm, that is of the human body and at which the ECG signal can be obtained through measurement.

S52. The first wearable device sends the blood pressure measurement instruction to the second wearable device.

Optionally, specified duration later after the blood pressure measurement instruction is sent, the first wearable device starts to perform blood pressure measurement. The specified duration is the same as duration in which the first wearable device sends the blood pressure measurement instruction to the second wearable device, to ensure that the first wearable device and the second wearable device simultaneously start to perform blood pressure measurement, so that blood pressure calculation accuracy is improved.

S53. The second wearable device receives the blood pressure measurement instruction sent by the first wearable device, where the blood pressure measurement instruction is used to instruct the second wearable device to perform blood pressure measurement, and the second wearable device is worn at a part that is of the human body and at which second blood pressure reference data can be obtained through measurement.

S54. The second wearable device sends feedback information to the first wearable device, where the feedback information is used to notify the first wearable device that the second wearable device successfully receives the blood pressure measurement instruction.

S55. The first wearable device receives the feedback information sent by the second wearable device, where the feedback information is used to notify the first wearable device that the second wearable device successfully receives the blood pressure measurement instruction.

Optionally, if the first wearable device does not receive, within a second specified time, the feedback information sent by the second wearable device, the first wearable device re-sends the blood pressure instruction to the second wearable device.

S56. The first wearable device starts to collect the first blood pressure reference data.

Specifically, the first wearable device starts to collect the first blood pressure reference data in the first specified duration.

S57. The second wearable device starts, third specified duration later after the feedback information is sent, to collect the second blood pressure reference data in a first specified duration.

Optionally, the third specified duration is set to equal to a time X consumed from sending of the feedback message by the second wearable device to receiving of the feedback message by the first wearable device, to make the second wearable device and the first wearable device simultaneously start to collect data.

For example, the first wearable device encapsulates a synchronization signal or a timestamp into a packet, where the packet further includes the blood pressure measurement instruction, and if Bluetooth is used to send the packet, a size of the packet is a maximum of 27 bytes; and sends the packet to the second wearable device. After receiving the packet, the second wearable device returns the feedback message indicating that the packet is successfully received. If the second wearable device fails to receive the packet, or the first wearable device does not receive the feedback message, the first wearable device re-encapsulates the packet, until the first wearable device successfully receives the feedback message, to complete synchronization or time calibration. For Bluetooth low energy (Bluetooth Low Energy, BLE), a time of the foregoing process may be preferably less than or equal to 10 ms (where a connection time interval is between 7.5 ms and 4s). For enhanced shock burst (Enhanced Shock Burst, ESB) of a dedicated 2.4 G protocol, a time of the foregoing process may be preferably less than or equal to 1 ms (a connection interval may be customized), and may be 460 µs through actual measurement.

S58. The second wearable device sends the second blood pressure reference data collected in the first specified duration to the first wearable device.

S59. The first wearable device receives the second blood pressure reference data that is collected by the second wearable device in the first specified duration.

Optionally, to ensure that a frequency offset delay Y of the first wearable device and the second wearable device falls within an allowable deviation range in the first specified duration, a specified temperature compensated crystal oscillator (Temperature Compensated Crystal Oscillator, TCXO) is selected for each of the first wearable device and the second wearable device to suppress a crystal oscillator frequency offset. Different TCXOs are corresponding to different crystal oscillator frequency offset delays. A TCXO whose frequency offset delay is 0.35 mm is used as an example in the following. If a sampling frequency is set to 1 KHz or 500 Hz, the first specified duration is 50s, a 1.5 ppm crystal oscillator is selected for the first wearable device, and a 5 ppm crystal oscillator is selected for the second wearable device, a frequency offset delay corresponding to 50s is 0.35 mm, and this meets a requirement that the frequency offset delay is less than 0.5 ms or 1 ms.

Preferably, the first specified duration is between 10s and 16s, and this is not limited in this embodiment of the present invention.

S510. The first wearable device calculates blood pressure based on the first blood pressure reference data that is collected by the first wearable device in the first specified duration and the second blood pressure reference data that is collected by the second wearable device in the first specified duration.

In this embodiment of the present invention, the blood pressure measurement method for a wearable device is provided. The first wearable device and the second wearable device simultaneously start to collect reference data at different parts of the human body, to calculate the blood pressure, so that blood pressure calculation accuracy is improved.

In a possible implementation, the first blood pressure reference data may be a photoplethysmogram PPG signal or an electrocardiograph ECG signal, and the second blood pressure reference data may be an ECG signal or a PPG signal; and when the first blood pressure reference data is a PPG signal, the second blood pressure reference data is an ECG signal; or when the first blood pressure reference data is an ECG signal, the second blood pressure reference data is a PPG signal.

In a possible implementation, the second blood pressure reference data may be a photoplethysmogram PPG signal or an electrocardiograph ECG signal.

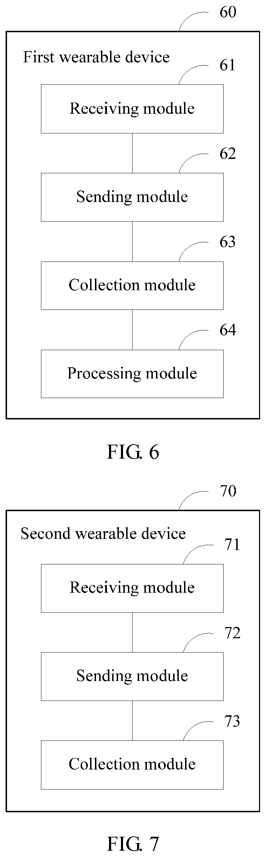

Based on a same inventive concept, an embodiment of the present invention provides a blood pressure measurement apparatus (a first wearable device) 60 for a wearable device. As shown in FIG. 6, the apparatus includes:
a receiving module 61, configured to generate a blood pressure measurement instruction, where the blood pressure measurement instruction is used to instruct a second wearable device to perform blood pressure measurement, and the first wearable device is worn at a part that is of a human body and at which first blood pressure reference data can be obtained through measurement;
a sending module 62, configured to generate the blood pressure measurement instruction to the second wearable device, where
the receiving module 61 is further configured to receive feedback information sent by the second wearable device, where the feedback information is used to notify the first wearable device that the second wearable device successfully receives the blood pressure measurement instruction;
a collection module 63, configured to collect the first blood pressure reference data based on the feedback information, where a time of collecting the first blood pressure reference data is first specified duration, where
the receiving module 61 is further configured to receive the second blood pressure reference data that is collected by the second wearable device in the first specified duration; and
a processing module 64, configured to calculate blood pressure based on the first blood pressure reference data and the second blood pressure reference data.

In this embodiment of the present invention, the blood pressure measurement apparatus for a wearable device is provided. The first wearable device and the second wearable device simultaneously start to collect reference data at different parts of the human body, to calculate the blood pressure, so that blood pressure calculation accuracy is improved.

Optionally, the first blood pressure reference data may be a photoplethysmogram PPG signal or an electrocardiograph ECG signal, and the second blood pressure reference data may be an ECG signal or a PPG signal; and
when the first blood pressure reference data is a PPG signal, the second blood pressure reference data is an ECG signal; or
when the first blood pressure reference data is an ECG signal, the second blood pressure reference data is a PPG signal.

Optionally, the sending module is further configured to: if the receiving module does not receive, within a second specified time, the feedback information sent by the second wearable device, re-send the blood pressure instruction to the second wearable device.

Optionally, the blood pressure measurement instruction generated by the receiving module may be triggered by a user, or may be triggered by the second wearable device.

Based on a same inventive concept, an embodiment of the present invention provides another blood pressure measurement apparatus (a second wearable device) 70 for a wearable device. As shown in FIG. 7, the apparatus includes:
a receiving module 71, configured to receive a blood pressure measurement instruction sent by a first wearable device, where the blood pressure measurement instruction is used to instruct the second wearable device to perform blood pressure measurement, and the second wearable device is worn at a part that is of a human body and at which second blood pressure reference data can be obtained through measurement;
a sending module 72, configured to send feedback information to the first wearable device, where the feedback information is used to notify the first wearable device that the receiving module successfully receives the blood pressure measurement instruction; and
a collection module 73, configured to start, third specified duration later after the sending module sends the feedback information, to collect the second blood pressure reference data in first specified duration, where
the sending module 72 is further configured to send the second blood pressure reference data collected in the first specified duration to the first wearable device.

Optionally, the second blood pressure reference data may be a photoplethysmogram PPG signal or an electrocardiograph ECG signal.

The following describes a structure and a processing manner of the apparatus in the embodiments of the present invention with reference to a preferred hardware structure.

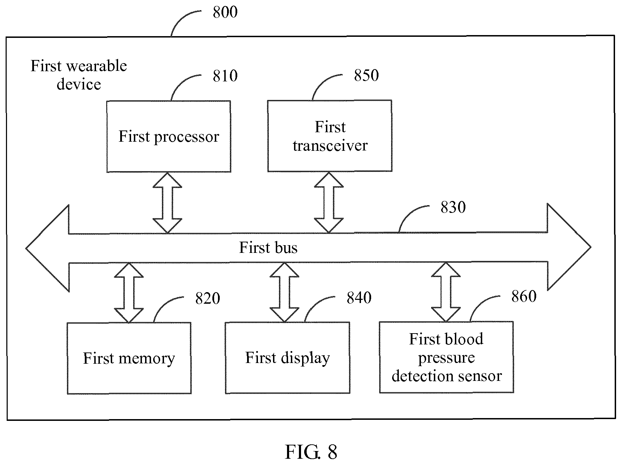

As shown in FIG. 8, an embodiment of the present invention provides a first wearable device 800 for measuring blood pressure, including a first processor 810, a first memory 820 connected to the first processor, a first display 840 that is connected to the first bus 830 and that is configured to display a blood pressure index, a first transceiver 850, and a first blood pressure detection sensor 860. The transceiver includes a first receiver and a first transmitter. The first processor 810, the first transceiver 850, the first memory 820, and the first blood pressure detection sensor 860 are mutually connected by using the first bus 830. The first processor generates a blood pressure measurement instruction based on a user operation or automatic setting. The first processor controls the first transmitter to send the blood pressure measurement instruction to a second wearable device.

The first receiver receives feedback information sent by the second wearable device, where the feedback information is used to notify the first wearable device that the second wearable device successfully receives the blood pressure measurement instruction.

The first processor controls, based on the feedback information received by the first receiver, the first blood pressure detection sensor to collect the first blood pressure reference data, where a time of collecting the first blood pressure reference data is first specified duration.

The first receiver is further configured to receive second blood pressure reference data that is collected by the second wearable device in the first specified duration.

The first processor calculates blood pressure based on the first blood pressure reference data and the second blood pressure reference data.

Optionally, the first blood pressure reference data may be a photoplethysmogram PPG signal or an electrocardiograph ECG signal, and the second blood pressure reference data may be an ECG signal or a PPG signal; and
when the first blood pressure reference data is a PPG signal, the second blood pressure reference data is an ECG signal; or
when the first blood pressure reference data is an ECG signal, the second blood pressure reference data is a PPG signal.

Optionally, if the first receiver does not receive, within a second specified time, the feedback information sent by the second wearable device, the first processor controls the first transmitter to re-send the blood pressure instruction to the second wearable device.

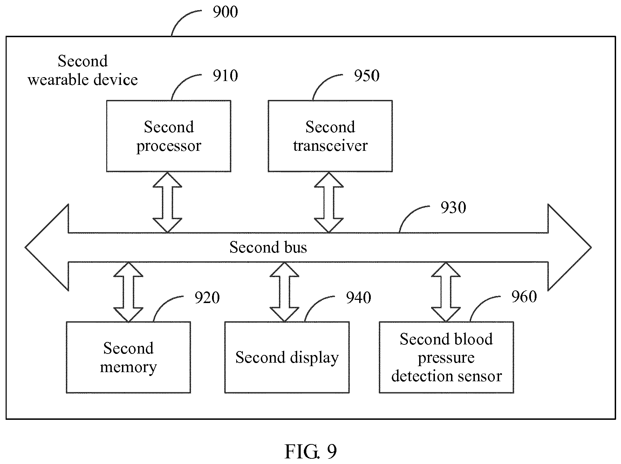

As shown in FIG. 9, an embodiment of the present invention provides a second wearable device 900 for measuring blood pressure, including a second processor 910, a second memory 920 connected to the second processor, a second display 940 that is connected to a second bus 930 and that is configured to display a quantity of steps, a second transceiver 950, and a second blood pressure detection sensor 960. The second transceiver includes a second receiver and a second transmitter. The second processor 910, the second transceiver 950, the second memory 920, and the second blood pressure detection sensor 960 are mutually connected by using the second bus 930. The second wearable device is worn at a part that is of a human body and at which second blood pressure reference data can be obtained through measurement. The second wearable device includes the second receiver, the second transmitter, the second blood pressure detection sensor, and the second processor. The second processor is connected to the second receiver, the second transmitter, and the second blood pressure detection sensor by using a data bus.

The second receiver receives a blood pressure measurement instruction sent by a first wearable device, where the blood pressure measurement instruction is used to instruct the second wearable device to perform blood pressure measurement, and the second wearable device is worn at a part that is of the human body and at which the second blood pressure reference data can be obtained through measurement.

The second transmitter sends feedback information to the first wearable device, where the feedback information is used to notify the first wearable device that the second wearable device receives the blood pressure measurement instruction.

The second processor controls the second blood pressure detection sensor to collect, third specified duration later after the second transmitter sends the feedback information, the second blood pressure reference data in first specified duration.

The second transmitter sends the second blood pressure reference data collected in the first specified duration to the first wearable device.

Optionally, the second blood pressure reference data may be a photoplethysmogram PPG signal or an electrocardiograph ECG signal.

Persons skilled in the art should understand that the embodiments of the present invention may be provided as a method, a system, or a computer program product. Therefore, the present invention may use a form of hardware only embodiments, software only embodiments, or embodiments with a combination of software and hardware. Moreover, the present invention may use a form of a computer program product that is implemented on one or more computer-usable storage media (including but not limited to a disk memory, a CD-ROM, an optical memory, and the like) that include computer-usable program code.

The present invention is described with reference to the flowcharts and/or block diagrams of the method, the device (system), and the computer program product according to the embodiments of the present invention. It should be understood that computer program instructions may be used to implement each process and/or each block in the flowcharts and/or the block diagrams and a combination of a process and/or a block in the flowcharts and/or the block diagrams. These computer program instructions may be provided for a general-purpose computer, a dedicated computer, an embedded processor, or a processor of any other programmable data processing device to generate a machine, so that the instructions executed by a computer or a processor of any other programmable data processing device generate an apparatus for implementing a specific function in one or more processes in the flowcharts and/or in one or more blocks in the block diagrams.

These computer program instructions may be stored in a computer readable memory that can instruct the computer or any other programmable data processing device to work in a specific manner, so that the instructions stored in the computer readable memory generate an artifact that includes an instruction apparatus. The instruction apparatus implements a specific function in one or more processes in the flowcharts and/or in one or more blocks in the block diagrams.

These computer program instructions may be loaded onto a computer or another programmable data processing device, so that a series of operations and steps are performed on the computer or the another programmable device, thereby generating computer-implemented processing. Therefore, the instructions executed on the computer or the another programmable device provide steps for implementing a specific function in one or more processes in the flowcharts and/or in one or more blocks in the block diagrams.

Although some preferred embodiments of the present invention have been described, persons skilled in the art can make changes and modifications to these embodiments once they learn the basic inventive concept. Therefore, the following claims are intended to be construed as to cover the preferred embodiments and all changes and modifications falling within the scope of the present invention.

Obviously, persons skilled in the art can make various modifications and variations to the present invention without departing from the spirit and scope of the present invention. The present invention is intended to cover these modifications and variations provided that they fall within the scope of protection defined by the following claims and their equivalent technologies.

## Claims

1. A blood pressure measurement method for a wearable device, wherein the method comprises:
generating, by a first wearable device, a blood pressure measurement instruction, wherein the blood pressure measurement instruction is used to instruct a second wearable device to perform blood pressure measurement, and the first wearable device is worn at a part that is of a human body and at which first blood pressure reference data can be obtained through measurement;
sending, by the first wearable device, the blood pressure measurement instruction to the second wearable device;
receiving, by the first wearable device, feedback information sent by the second wearable device, wherein the feedback information is used to notify the first wearable device that the second wearable device successfully receives the blood pressure measurement instruction;
collecting, by the first wearable device, the first blood pressure reference data based on the feedback information, wherein a time of collecting the first blood pressure reference data is first specified duration;
receiving, by the first wearable device, second blood pressure reference data that is collected by the second wearable device in the first specified duration; and
calculating, by the first wearable device, blood pressure based on the first blood pressure reference data and the second blood pressure reference data.

2. The method according to claim 1, wherein the first blood pressure reference data may be a photoplethysmogram PPG signal or an electrocardiograph ECG signal, and the second blood pressure reference data may be an ECG signal or a PPG signal; and
when the first blood pressure reference data is a PPG signal, the second blood pressure reference data is an ECG signal; or
when the first blood pressure reference data is an ECG signal, the second blood pressure reference data is a PPG signal.

3. The method according to claim 1, wherein the method further comprises: if the first wearable device does not receive, within a second specified time, the feedback information sent by the second wearable device, re-sending, by the first wearable device, the blood pressure instruction to the second wearable device.

4. The method according to claim 1, wherein the blood pressure measurement instruction generated by the first wearable device may be triggered by a user, or may be triggered by the second wearable device.

5. A blood pressure measurement method for a wearable device, wherein the method comprises:
receiving, by the second wearable device, a blood pressure measurement instruction sent by a first wearable device, wherein the blood pressure measurement instruction is used to instruct the second wearable device to perform blood pressure measurement, and the second wearable device is worn at a part that is of a human body and at which second blood pressure reference data can be obtained through measurement;
sending, by the second wearable device, feedback information to the first wearable device, wherein the feedback information is used to notify the first wearable device that the second wearable device successfully receives the blood pressure measurement instruction;
starting, by the second wearable device third specified duration later after the feedback information is sent, to collect the second blood pressure reference data in a first specified duration; and
sending, by the second wearable device, the second blood pressure reference data collected in the first specified duration to the first wearable device.

6. The method according to claim 5, wherein the second blood pressure reference data may be a photoplethysmogram PPG signal or an electrocardiograph ECG signal.

7. A blood pressure measurement apparatus for a wearable device, wherein the apparatus comprises:
a receiving module, configured to generate a blood pressure measurement instruction, wherein the blood pressure measurement instruction is used to instruct a second wearable device to perform blood pressure measurement, and the first wearable device is worn at a part that is of a human body and at which first blood pressure reference data can be obtained through measurement;
a sending module, configured to send the blood pressure measurement instruction to the second wearable device, wherein
the receiving module is further configured to receive feedback information sent by the second wearable device, wherein the feedback information is used to notify the first wearable device that the second wearable device successfully receives the blood pressure measurement instruction;
a collection module, configured to collect the first blood pressure reference data based on the feedback information, wherein a time of collecting the first blood pressure reference data is first specified duration, wherein
the receiving module is further configured to receive the second blood pressure reference data that is collected by the second wearable device in the first specified duration; and
a processing module, configured to calculate blood pressure based on the first blood pressure reference data and the second blood pressure reference data.

8. The apparatus according to claim 7, wherein the first blood pressure reference data may be a photoplethysmogram PPG signal or an electrocardiograph ECG signal, and the second blood pressure reference data may be an ECG signal or a PPG signal; and
when the first blood pressure reference data is a PPG signal, the second blood pressure reference data is an ECG signal; or
when the first blood pressure reference data is an ECG signal, the second blood pressure reference data is a PPG signal.

9. The apparatus according to claim 7, wherein the sending module is further configured to: if the receiving module does not receive, within a second specified time, the feedback information sent by the second wearable device, re-send the blood pressure instruction to the second wearable device.

10. The apparatus according to claim 7, wherein the blood pressure measurement instruction generated by the receiving module may be triggered by a user, or may be triggered by the second wearable device.

11. A blood pressure measurement apparatus for a wearable device, wherein the apparatus comprises:
a receiving module, configured to receive a blood pressure measurement instruction sent by a first wearable device, wherein the blood pressure measurement instruction is used to instruct the second wearable device to perform blood pressure measurement, and the second wearable device is worn at a part that is of a human body and at which second blood pressure reference data can be obtained through measurement;
a sending module, configured to send feedback information to the first wearable device, wherein the feedback information is used to notify the first wearable device that the receiving module successfully receives the blood pressure measurement instruction; and
a collection module, configured to start, third specified duration later after the sending module sends the feedback information, to collect the second blood pressure reference data in first specified duration, wherein
the sending module is further configured to send the second blood pressure reference data collected in the first specified duration to the first wearable device.

12. The apparatus according to claim 11, wherein the second blood pressure reference data may be a photoplethysmogram PPG signal or an electrocardiograph ECG signal.

13. A first wearable device for measuring blood pressure, wherein the first wearable device is worn at a part that is of a human body and at which first blood pressure reference data can be obtained through measurement, the first wearable device comprises a first receiver, a first transmitter, a first blood pressure detection sensor, and a first processor, and the first processor is connected to the first receiver, the first transmitter, and the first blood pressure detection sensor by using a bus, wherein
the first processor generates a blood pressure measurement instruction based on a user operation or automatic setting, and the first processor controls the first transmitter to send the blood pressure measurement instruction to a second wearable device;
the first receiver receives feedback information sent by the second wearable device, wherein the feedback information is used to notify the first wearable device that the second wearable device successfully receives the blood pressure measurement instruction;
the first processor controls, based on the feedback information received by the first receiver, the first blood pressure detection sensor to collect the first blood pressure reference data, wherein a time of collecting the first blood pressure reference data is first specified duration;
the first receiver is further configured to receive second blood pressure reference data that is collected by the second wearable device in the first specified duration; and
the first processor calculates blood pressure based on the first blood pressure reference data and the second blood pressure reference data.

14. The first wearable device according to claim 13, wherein the first blood pressure reference data may be a photoplethysmogram PPG signal or an electrocardiograph ECG signal, and the second blood pressure reference data may be an ECG signal or a PPG signal; and
when the first blood pressure reference data is a PPG signal, the second blood pressure reference data is an ECG signal; or
when the first blood pressure reference data is an ECG signal, the second blood pressure reference data is a PPG signal.

15. The first wearable device according to claim 13, wherein if the first receiver does not receive, within a second specified time, the feedback information sent by the second wearable device, the first processor controls the first transmitter to re-send the blood pressure instruction to the second wearable device.

16. A second wearable device for measuring blood pressure, wherein the second wearable device is worn at a part that is of a human body and at which second blood pressure reference data can be obtained through measurement, the second wearable device comprises a second receiver, a second transmitter, a second blood pressure detection sensor, and a second processor, and the second processor is connected to the second receiver, the second transmitter, and the second blood pressure detection sensor by using a data bus, wherein
the second receiver receives a blood pressure measurement instruction sent by a first wearable device, wherein the blood pressure measurement instruction is used to instruct the second wearable device to perform blood pressure measurement, and the second wearable device is worn at a part that is of the human body and at which the second blood pressure reference data can be obtained through measurement;
the second transmitter sends feedback information to the first wearable device, wherein the feedback information is used to notify the first wearable device that the second wearable device receives the blood pressure measurement instruction;
the second processor controls the second blood pressure detection sensor to collect, third specified duration later after the second transmitter sends the feedback information, the second blood pressure reference data in first specified duration; and
the second transmitter sends the second blood pressure reference data collected in the first specified duration to the first wearable device.

17. The second wearable device according to claim 16, wherein the second blood pressure reference data may be a photoplethysmogram PPG signal or an electrocardiograph ECG signal.
